# EUROPEAN PATENT APPLICATION

(11) **EP 2 565 645 A1**
(43) Date of publication of application: **06.03.2013**
(21) Application number: 11179783.3
(22) Date of filing: 01.09.2011
(51) Int. Cl.: G01N 33/00, G01N 27/12, B82Y 15/00

(54) **Fluid detector and method for detecting fluids**

(71) Applicant: Universität zu Köln, 50923 Köln (DE); Universitat de Barcelona, 08028 Barcelona (ES); Fundacio Institut Recerca en Energia de Catalunya, 08930 Barcelona (ES)
(72) Inventor: Shen, Hao, 50674 Köln (DE); Mathur, Sanjay, 50859 Köln (DE); Hoffmann, Martin Wolfgang Georg, 50321 Brühl (DE); Gad, Alaa Eldin Abd Eltawab Mohamed, 50939 Köln (DE); Prades Garcia, Juan Daniel, E08004 Barcelona (ES); Hernandez-Ramirez, Francisco, E08901 L'Hospitalet de Llobregat (ES)
(74) Representative: Michalski Hüttermann & Partner Patentanwälte

(57) **Abstract**

The present invention relates to a fluid detector (10) comprising at least one semiconductor detector cell (12), the semiconductor detector cell (12) including:
- a photovoltaic semiconductor device (14) with at least one portion (16) made of a first semiconducting type material and at least one other portion (18) made of a second semiconducting type material, wherein the first and second semiconducting type materials differ in their charge carrier types and/or the density of their charge carriers and wherein the different portions (16, 18) adjoin each other with the formation of a junction between said different charge carrier types and/or charge carrier densities; and
- a system for photo-induced electron transfer, the system comprising a plurality of semiconducting nanoparticles (28) on at least one surface area (30) of the portion (16) made of the second semiconducting type material, wherein said surface area (30) is accessible for the fluid (32).

The invention further relates to a corresponding detecting method.

## Description

The invention relates to a fluid detector, in particular a gas detector, comprising at least one semiconductor detector cell. The invention further relates to a method for detecting fluids, in particular gas atmospheres and their mixtures.

A gas detector or gas sensor is a device, which detects the presence of various gases within an area, usually as part of a safety system. This type of equipment is used to detect a gas leak and interface with a control system so a process can be automatically shut down. Gas detectors are usually operated with battery.

Semiconductor detectors detect gases by a chemical reaction that takes place when the gas comes in contact with the detector cell. Metal oxide semiconductors are the most common materials used in cells of semiconductor detectors, and the electrical resistance in the detector changes when it is exposed to the monitored gas. The change in resistance is used to calculate the gas concentration. Semiconductor detectors are commonly used to detect hydrogen, oxygen, alcohol, and harmful gases such as carbon monoxide etc.

It is an object of the invention to provide a simple and efficient fluid detector and a simple and efficient method for detecting fluids.

This object is achieved by the independent claims. The dependent claims detail advantageous embodiments of the invention.

According to the invention, the semiconductor detector cell of the fluid detector includes a photovoltaic semiconductor device with at least one portion made of a first semiconducting type material and at least one other portion made of a second semiconducting type material. The first and second semiconducting type materials differ from one another in their charge carrier types and/or the density of their charge carriers, wherein the different portions adjoin each other with the formation of a junction between said different charge carrier types and/or charge carrier densities. The semiconductor detector cell further includes a system for photo-induced electron transfer, the system comprising a plurality of semiconducting nanoparticles (NPs) arranged on at least one surface area of the portion made of the second semiconducting type material, wherein said surface area is accessible for the fluid. The photovoltaic effect is a generation of a voltage or a corresponding electric current in a material upon exposure to light.

The light for driving the photovoltaic semiconductor device can be light in the visible spectrum, the near infrared (near IR) spectrum and the near ultraviolet (near UV) spectrum. Preferably, the light for driving the photovoltaic semiconductor device is light with photon energy below the near UV range. Therefore, the corresponding photovoltaic semiconductor preferably is a photovoltaic semiconductor suitable for the use with light having photon energy below the near UV range. The visible spectrum is the portion of the electromagnetic spectrum that is visible to the human eye (wavelength range of 400 - 760 nm corresponding to 3.1 - 1.6 eV photon energy). The wavelength range of near UV is 300 - 400 nm (corresponding to 4.1 - 3.1 eV photon energy). The wavelength range of near IR is 760 - 3000 nm (corresponding to 1.6 - 0.4 eV photon energy). Within these semiconductors the junction is needed for establishing a built-in potential capable of driving the photovoltaic effect, especially a charge separation of the charge carriers, which are set free when light provides them with adequate energy.

In general, the fluid can be a gas, a liquid, an aerosol, and a plasma, etc. Preferably, the fluid is a gas or gaseous mixture and the fluid detector is a gas detector.

The main idea of the fluid detector is a combination of a photovoltaic semiconductor device for generating an output signal (the voltage or the corresponding electric current) of the detector driven by the energy input of incident light/photons, and a system for photo-induced electron transfer to the at least one surface area of said semiconductor device. This surface area becomes an active surface area by means of the electron transfer. The interaction of fluid molecules and/or fluid atoms with the active surface area changes the output signal depending on a quantity of fluid molecules and/or fluid atoms interacting with the active surface area of the detector. The interaction is a transfer of electrons in terms of reduction/oxidation. The activation process to obtain the at least one active surface area is driven by the energy input of incident light/photons as well.

By use of a suitable combination of materials, the light from the same light source can drive the process for generating the output signal of the photovoltaic semiconductor device and the activation process of the surface area by means of the photo-induced electron transfer. Therefore, the only energy source for generating a measurable output signal is the incident light or photons.

The nanoparticles and the portion of the semiconductor device made of the first semiconducting type material have a particular energy gap between the energy levels of their valence and conduction electrons. Preferably, the energy gap of nanoparticles is smaller than the corresponding energy gap of the semiconductor diode portion made of the first semiconducting type material. In this case, the work function of said first semiconducting type material is higher than the work function of the nanoparticle material, namely at least by the amount of the difference between the two energy gaps. The general idea of photo-induced electron transfer (or photo-induced charge transfer) and several combinations of materials suitable for this transfer are known. The document »Tvrdy, K. et al.: "Photoinduced electron transfer from semiconductor quantum dots to metal oxide nanoparticles"; Proceedings of the National Academy of Sciences, vol. 108, issue 1, pp. 29-34 (2011)« describes for example the electron transfer in nanoparticulate systems with semiconducting quantum dots (QD) and metal oxide (MO) semiconducting nanostructures.

According to preferred embodiments of the present invention, the junction between said different charge carrier types and/or charge carrier densities is a p-n junction or p-i-n junction.

According to another preferred embodiment of the invention, the photovoltaic semiconductor device is designed as a photodiode or a photoelectric cell. A photodiode is a semiconductor diode for converting light (visible light, infrared light, ultra-violet light and/or X-ray) into an electric current by means of the photovoltaic effect. A photoelectric cell (also called solar cell or photovoltaic cell) is a solid-state electrical device that converts the energy of light directly into electricity by the photovoltaic effect.

The fluid detector is a light-driven fluid detector that can be operated at room temperature. The light for driving the photovoltaic semiconductor device can be solar light. Under solar illumination, the at least one detector cell of the detector generates an open circuit voltage V_{oc} that can be used as detecting signal, eliminating the need of any external electric power source. This open circuit voltage V_{oc} is the output signal of the detector generated by the semiconductor device being a photodiode or a photoelectric cell.

According to other embodiments, the nanoparticles are light absorbing dots usable for light in the visible spectrum. By means of the absorption process, each nanoparticle generates electron-hole pairs. The referring excited electrons can transfer to the valence band of the portion made of the first semiconducting type material in the surface area. This electron injection process (from nanoparticles to the second semiconducting type material) locally modifies the electron concentration in said surface area. These electrons can interact with non-inert fluids at the said surface area.

The light for driving the complete fluid detector can be solar light. Therefore, the fluid detector is a self-powered fluid detector without a demand for an external power source or battery to drive the detector.

According to yet other embodiments, the portion made of the first semiconducting type material forms a substrate on which the portion made of the second semiconducting type material is built as a structured portion, especially a nano-structured portion. Preferably, the at least one portion made of the first semiconducting type material is structured as a nanorod (NR).

The first semiconducting type material is suitable to establish a built-in potential with the second semiconducting type material. Preferably, the first semiconducting type material is n-doped Silicon (n-Si) or p-doped Silicon (p-Si).

According to other preferred embodiments, the first semiconducting type material is a p-type metal oxid or an n-type metal oxide (MOₓ). Especially, the n-type or p-type metal oxide is one of the following materials:
- n-type Zinc oxide (n-ZnO),
- n-type Titanium dioxide (n-TiO₂),
- n-type Tin dioxide (n-SnO₂),
- n-type Tungsten trioxide (n-WO₃),
- n-type Niobium oxide (NbOₓ),
- n-type Vanadium oxide (VOₓ),
- n-type Indium(III) oxide (In₂O₃),
- n-type Iron(III) oxide (Fe₂O₃),
- p-type Nickel(II) oxide (p-NiO),
- p-type Copper(II) oxide (p-CuO), and
- bimetallic oxides (e.g. CuAlO₂ or CuAl₂O₄).

Advantageously, the photovoltaic semiconductor device of the at least one detector cell is an n-metal oxide//p-Si or p-metal oxide//n-Si solar cell.

According to other preferred embodiments, the nanoparticles are made of one of the following materials:
- Cadmium sulfide (CdS),
- Cadmium selenide (CdSe),
- Cadmium telluride (CdTe),
- Lead(II) sulfide (PbS),
   Indium(III) phosphide (InP),
   Tin(II) sulfide (SnS),
   Tin(IV) sulfide (SnS₂),
   Bismuth(III) sulfide (Bi₂S₃),
- Copper(II) oxide (CuO), and
- other sensitizers such as dyes.

Preferably, the fluid detector comprises a plurality of detector cells, said detector cells being electrically interconnected in parallel and/or in series with each other. Especially, at least one pair of portions made of the first semiconducting type material are of a one-piece construction forming a single structured portion of two interconnected detector cells.

According to other preferred embodiments, the detector further comprises electrodes for contacting the at least one semiconductor detector cell of the fluid detector. Preferably, at least one of the electrodes is transparent for visible light (transparent electrode). This transparent electrode can be a transparent conducting film of inorganic or organic materials. Inorganic films typically are made up of a layer of TCO (transparent conducting oxide), generally in the form of indium tin oxide (ITO), fluorine doped tin oxide (FTO), and doped zinc oxide. Organic films are being developed using carbon nano-tube networks and graphene, which can be fabricated to be highly transparent to the infrared light, along with networks of polymers such as poly(3,4-ethylenedioxythiophene) and its derivatives.

The present invention further refers to a method for detecting fluids, especially gases, by means of a fluid detector. The fluid detector comprises at least one semiconductor detector cell including a photovoltaic semiconductor device with at least one portion made of a first semiconducting type material and at least one other portion made of a second semiconducting type material. The first and second semiconducting type materials differ in their charge carrier types and/or the density of their charge carriers, wherein the different portions adjoin each other with the formation of a junction between said different charge carrier types and/or charge carrier densities. The semiconductor detector cell further includes a system for photo-induced electron transfer, the system comprising a plurality of semiconducting nanoparticles on at least one surface area of the portion made of the second semiconducting type material, which surface area is accessible for the fluid. The method comprises the steps of (i) illuminating the fluid detector with light having a photon energy below the near UV range and (ii) measuring a voltage and/or a current of the illuminated semiconductor detector cell. The fluid detector preferably is an aforementioned fluid detector.

The photovoltaic semiconductor device of the fluid detector preferably is a photodiode or a photoelectric cell. The measuring of the corresponding voltage and/or current is a measuring of an open circuit voltage V_{oc} of the illuminated semiconductor detector cell.

These and other aspects of the invention will be apparent from and elucidated with reference to the embodiments described hereinafter.

In the drawings:
Fig. 1 depicts a schematic representation of a fluid detector with one semiconductor detector cell according to a first preferred embodiment of the invention,
Fig. 2 depicts a schematic representation of a photovoltaic semiconductor device and a nanoparticle of the detector cell depicted in Fig. 1,
Fig. 3 shows the corresponding energy levels of a first portion of the photovoltaic semiconductor device and of the nanoparticle,
Fig. 4 shows a detector signal of the fluid detector recorded by switching between an oxigen- and a nitrogen-surrounding atmosphere,
Fig. 5 shows a detector signal of the fluid detector recorded by switching between an ethanol- (50, 100 and 200 ppm pulses) and a air-surrounding atmosphere, and
Figs. 6 to 9 show the manufacturing steps of the construction of a fluid detector with a plurality of detector cells according to a second preferred embodiment of the invention.

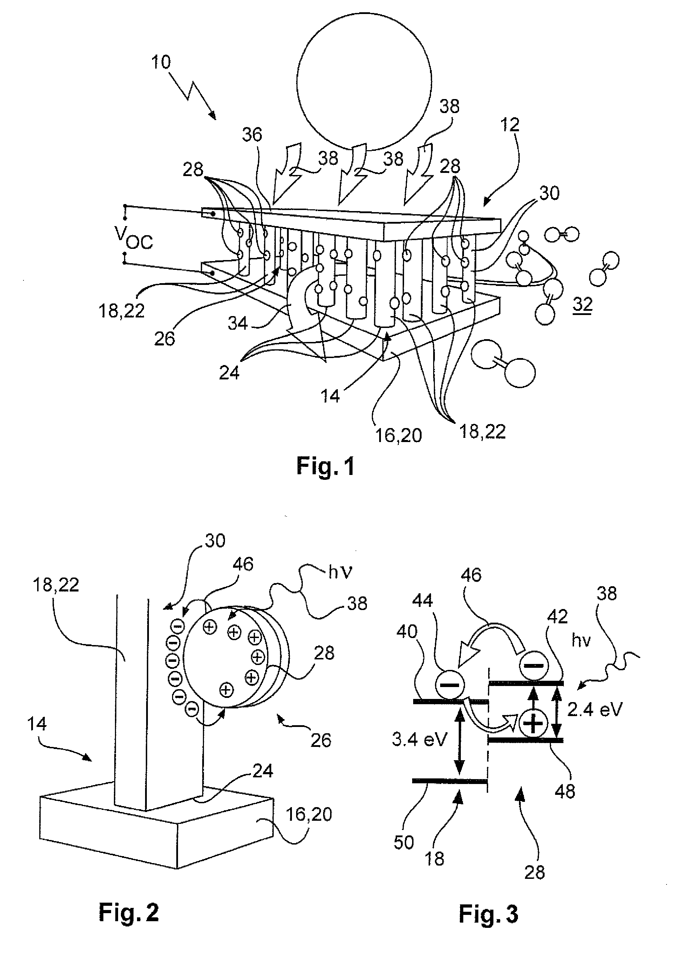

Fig. 1 shows a fluid detector 10, comprising a single semiconductor detector cell 12. The semiconductor detector cell 12 includes a photovoltaic semiconductor device 14 with a portion made of a first semiconducting type material 16 and a plurality of portions made of a second semiconducting type material 18. The portion made of the first semiconducting type material 16 forms a substrate 20 on which the portions made of the second semiconducting type material 18 are build as structured portions 22, especially nano-structured portions. The structured portions 22 shown in Fig. 1 are nanorods. The portions made of the second semiconducting type material 18 adjoin the upper side of the portion made of the first semiconducting type material 16 with the formation of p-n junctions at the border areas 24 between the two types of portions 16, 18.

The semiconductor detector cell 12 further comprises a system 26 for photo-induced electron transfer. The system 26 comprises a plurality of semiconducting nanoparticles 28 arranged on respective surface areas 30 of the portions made of the second semiconducting type material 18. Each of said surface areas 30 with the nanoparticles 28 is accessible for the fluid 32. Beneath some fluid elements, Fig. 1 shows a fluid stream (arrow 34) through the arrangement of the structured portions 22. In general, the fluid 32 can be a gas, a liquid, an aerosol, a plasma, etc. Preferably, the fluid is a gas or gaseous mixture and the fluid detector 10 is a gas detector.

The structured portions 22 are formed as nanorods extending perpendicular to the surface of the substrate 20. An electrode is mounted on their upper side of these nanorods. This electrode is a transparent electrode 36 allowing an illumination of the detector cell 12. The transparent electrode 36 is a thin film electrode, especially fluorine doped tin oxide (FTO) electrode, wherein the thin film is substantially aligned in plane-parallel relationship to the surface of the substrate 20.

The photovoltaic semiconductor device 14 shown in Fig. 1 is an n-metal oxide/p-Si solar cell, wherein the n-metal oxide (n-MO) is n-type Zinc oxide (n-ZnO). The material of the nanoparticles (NPs) 28 is Cadmium sulfide (CdS). The detector cell 12 shown in Fig. 1 is a CdS@n-ZnO/p-Si detector cell.

The energy input of the incident solar light (arrows 38) drives the photovoltaic semiconductor device 14 with the two types of portions 16, 18 as well as the system 26 for photo-induced electron transfer from the nanoparticles 28 to the at least one surface area of said semiconductor detector device 12.

Fig. 2 shows details of both main elements of the detector device 12. One part of the photovoltaic semiconductor device 14 with the portion 16 made of a first semiconducting type material and one of the other portions 18 made of a second semiconducting type material, wherein both portions 16, 18 form of a p-n junction; and one part of the system for photo-induced electron transfer 26, which system 26 comprises the plurality of semiconducting nanoparticles 28 on the surface area 26 of the portion 18 made of the second semiconducting type material.

Fig. 3 shows the corresponding energy levels of the portion 18 of the second semiconducting type and of the nanoparticle 28 on the surface area 30 of said portion 18 made of the second semiconducting type material.

The nanoparticles create electron-hole pairs upon absorption photons (right side). The proper band alignment between the material of the first semiconducting type (e.g. n-ZnO) and the material of the nanoparticles (e.g. CdS), in which the conduction band 40 edge of the material of the second semiconducting type is lower than the conduction band edge 42 of material of the nanoparticle 28, favours an effective exciton separation. Excited electrons (e*⁻) 44 are injected from the conduction band 42 of the material of the nanoparticles into the conduction band 40 of material of the second semiconducting type (arrow 46), whilst the mismatch in the valence band 48 forces holes to remain in the material of the nanoparticles. The energy gap between the valence band 48 edge and the conduction band 42 edge of the CdS is about 2.4 eV. The energy gap between the valence band 50 edge and the conduction band 40 edge of the n-ZnO is about 3.4 eV. The photon energy hv of the incident light 38 must be above a threshold given by the energy gap between the valence band 48 edge and the conduction band 42 edge of the nanoparticle material. In the case of CdS, the threshold is above 2.4 eV.

With ongoing injection of electrons (arrow 38), the number of holes within the material of the nanoparticles increases and an electrical field between the positive (located at the material of the nanoparticles) and negative charges/electrons 44 (at the material of the first semiconducting type) is formed. This self-limiting process continues until the electric field blocks any further injections of electrons. The result is a steady state with an overall increased electron density in the surface area 30 of the material of the second semiconducting type 18 that accumulates around the material of the nanoparticles 28 on the surface area 30 of the material of the second semiconducting type 18. Consequently, the dynamic processes of electron injection (from the material of the nanoparticles to the material of the second semiconducting type) and recombination (from the material of the second semiconducting type to the material of the nanoparticles) ends up in a steady state in which an overall modified electron density on the surface area 30 of the material of the first semiconducting type occurs.

The interaction of fluid molecules and/or fluid atoms with the (active) surface area 30 changes the output signal V_{oc} depending on a quantity of fluid molecules and/or fluid atoms interacting with the active surface area 30 of the detector cell 12. The interaction is a transfer of electrons 44 in terms of reduction/oxidation. The activation process to obtain the at least one surface area 30 is driven by the energy input of incident light/photons (arrow 38) as well.

Growth of the photovoltaic semiconductor device 14: The vertically oriented ZnO nanorod array shown in Figs. 1 and 2 was grown on a ZnO seed layer (20 nm) coated p-Si substrate. Firstly the Zn film was prepared by DC sputtering using Zn (99.99 %) target and high purity Ar (99.999 %) gas and then annealed in air at 500 °C for 1 h to obtain the crystalline ZnO seed layer. The array of ZnO nanorods was grown on the substrate 20 using a hydrothermal synthesis. Briefly, the growth solution was composed of zinc nitrate hexahydrate (0.025 mol L⁻¹), hexamethylenetetramine (0.025 mol L⁻¹), and aluminum-iso-propylate (0.005 mol L⁻¹). The temperature of the growth solution was maintained at 75 °C in a water tank. The pH value of the growth solution was monitored at 7.2 during the growth process. After 20 h, the products on the substrate were washed with deionised water several times and annealed in air at 400 °C for 1 h.

Growth of the system for photo-induced electron transfer 26: CdS nanoparticles were deposited on the surface of the ZnO nanorods via a chemical bath deposition (CBD). 2 mmol CdS precursor solution was prepared by dissolving CdSO₄ and thiourea (molar ratio urea: Cd²⁺ = 5:1) in 1 mol L⁻¹ ammonia solution. Before the decoration reaction the ZnO nanorod arrays were cleaned in a N₂ flow and then immersed into the CdS precursor solution for several minutes to ensure a complete diffusion of the precursor into the voids among ZnO nanorods. The solution containing the ZnO substrate was heated in an oil bath at 60 °C. During the CBD process, the sample color was changed from colorless to bright yellow. The deposition time for CdS nanoparticles was 15 minutes. After the CBD process, the ZnO samples were immediately washed with deionised water and ethanol.

A reproducible response (change of V_{oc}) towards oxidizing (oxygen) and reducing gases (ethanol, methane) is obtained. By use of nanoparticles/nanorods charge transfer interactions induced by the incident light (arrow 38) with a photon energy hv, gas-material surface interactions and the subsequent changes of charge carrier concentration (N_{D}), that turn into variation of V_{oc} in the semiconductor detector cell 12.

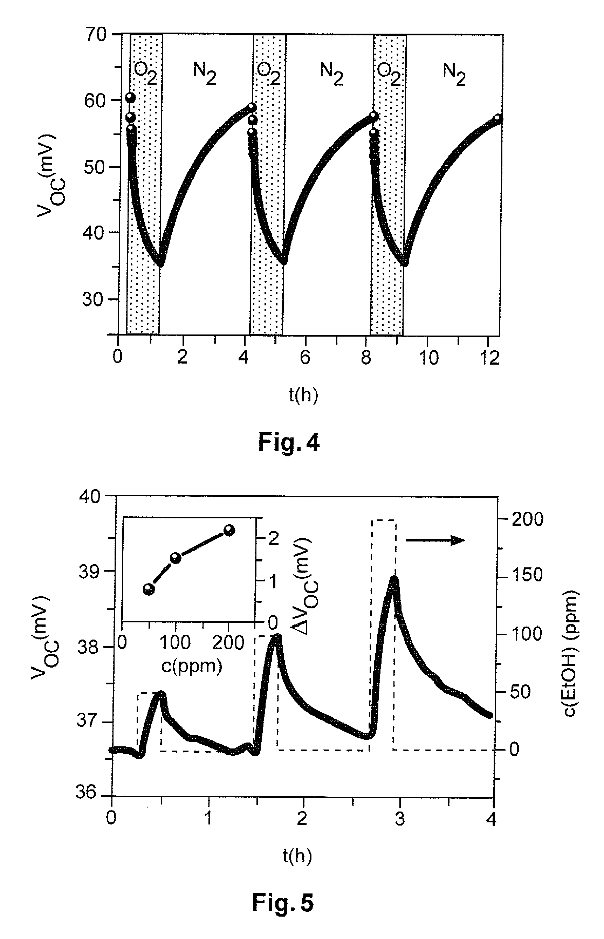

Fig. 4 represents an open circuit voltage (V_{oc}) vs. time (t) graph of an output detector signal of the semiconductor detector cell 12 (CdS@n-ZnO/p-Si detector cell). The graph is recorded by switching between an oxygen (O₂-) and a nitrogen (N₂-) atmosphere surrounding the detector cell 12.

Fig. 5 shows a detector signal of the semiconductor detector cell 12 recorded by switching between an ethanol (EtOH) surrounding atmosphere (50, 100 and 200 ppm pulses) and an air-surrounding atmosphere.

As illustrated in the graphs of Figs. 4 and 5, reproducible detector signals representing the presence of non-inert gases (like O₂) and inert gases (like N₂, noble gas or mixtures thereof) can be produced with the aforementioned fluid detector 10 based on a CdS@n-ZnO/p-Si detector cell.

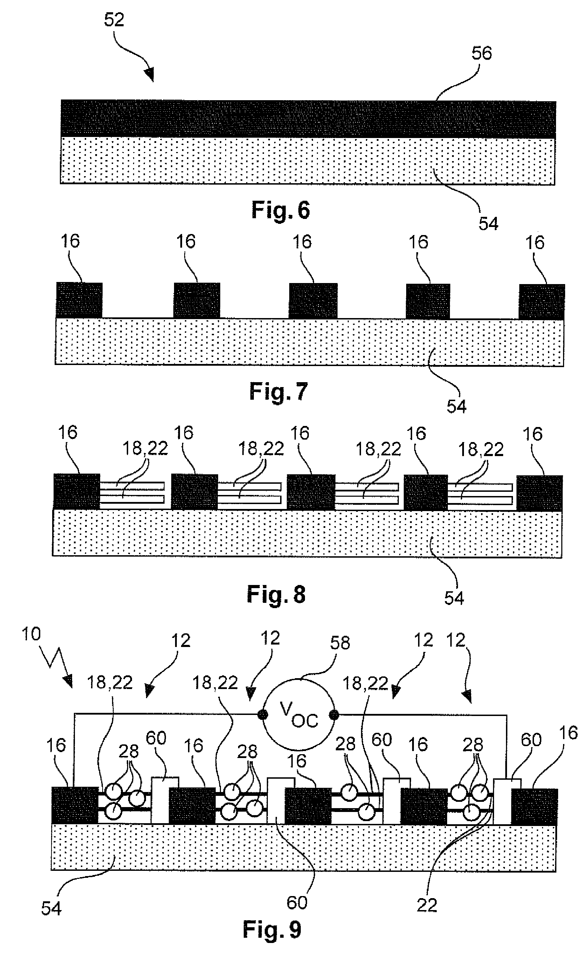

Figs. 6 to 9 show the steps of a manufacturing of a fluid detector with a plurality of detector cells according to a second preferred embodiment of the invention.

Fig. 6 shows a first step of providing a substrate system 52 being a layer system with an insulating base layer 54 and a semiconductor layer 56 made of the first semiconducting type material (e.g. p-Si).

In a second step, a photolithography- and etching-process structures the semiconductor layer 56. In this structuring step, the semiconductor layer 56 is divided into a plurality of portions 16 of the first semiconducting type on the insulating base layer 54. Fig. 7 shows the resulting system with the structured semiconductor comprising the separated portions 16 on the insulating base layer 54.

In a third step, a seeding material (not shown) is applied to the sidewalls of the portions 16 of the first semiconducting type. Afterwards the material of the second semiconducting type material is grown on one sidewall of each portion 16 of the first semiconducting type material to create rod-shaped portions 22 of the second semiconducting type material.

In a photolithographic forth step, the surface areas 26 of the rod-like portions 22 made of the second semiconducting type material are decorated by a plurality of nanoparticles 28. Further on, electrodes are applied to selected portions 16 of the first semiconducting type to complete the interlink connections of the rod-shaped portions 22 between the portions 16 of the first semiconducting type material. Preferably, these electrodes are metallic electrodes 60. Fig. 9 shows the resulting fluid detector 10 comprises the plurality of semiconductor detector cells 12, wherein said semiconductor detector cells 12 are electrically interconnected in series with each other via the electrodes 36. With the completed interlinks, a stack of semiconductor detector cells 12 is created. Fig. 8 shows the resulting array of detector cells 12. The fluid detector 10 further comprises a measuring device 58 for measuring a voltage of the semiconductor detector cells 12.

While the invention has been illustrated and described in detail in the drawings and foregoing description, such illustration and description are to be considered illustrative or exemplary and not restrictive; the invention is not limited to the disclosed embodiments.

Other variations to be disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting scope.

### List of Reference Signs:

- 10: fluid detector
- 12: detector cell
- 14: photovoltaic semiconductor device
- 16: portion (1^{st} semiconducting type material)
- 18: portion (2^{nd} semiconducting type material)
- 20: substrate
- 22: structured portion
- 24: border area
- 26: system for electron transfer
- 28: nanoparticle
- 30: surface area
- 32: fluid
- 34: arrow (fluid stream)
- 36: transparent electrode
- 38: arrow (light)
- 40: conduction band edge
- 42: conduction band edge
- 44: electron
- 46: arrow (transfer)
- 48: valence band edge
- 50: valence band edge
- 52: substrate system
- 54: insulating base layer
- 56: semiconductor layer
- 58: measuring device
- 60: metallic electrode

## Claims

1. Fluid detector (10) comprising at least one semiconductor detector cell (12), the semiconductor detector cell (12) including:
- a photovoltaic semiconductor device (14) with at least one portion (16) made of a first semiconducting type material and at least one other portion (18) made of a second semiconducting type material, wherein the first and second semiconducting type materials differ in their charge carrier types and/or the density of their charge carriers and wherein the different portions (16, 18) adjoin each other with the formation of a junction between said different charge carrier types and/or charge carrier densities; and
- a system for photo-induced electron transfer, the system comprising a plurality of semiconducting nanoparticles (28) on at least one surface area (30) of the portion (16) made of the second semiconducting type material, wherein said surface area (26) is accessible for the fluid (32).

2. Fluid detector according to claim 1, wherein the junction between said different charge carrier types and/or charge carrier densities is a p-n junction or p-i-n junction.

3. Fluid detector according to claim 1 or 2, wherein the photovoltaic semiconductor device (14) is designed as a photodiode or photoelectric cell.

4. Fluid detector according to one of claims 1 to 3, wherein the nanoparticles (28) are made of a narrow band gap semiconducting material absorbing light with a photon energy below the near UV range.

5. Fluid detector according to one of claims 1 to 4, wherein the portion (18) made of the first semiconducting type material forms a substrate (20) on which the portion (16) made of the second semiconducting type material is built as a structured portion (22), especially a nano-structured portion.

6. Fluid detector according to claim 5, wherein the at least one portion (16) made of the second semiconducting type material or at least the surface area (30) of said portion (16) is structured as a nanorod and/or nanowire and/or other nano-scaled morphologies.

7. Fluid detector according to one of claims 1 to 6, wherein the first semiconducting type material is n-doped Silicon or p-doped Silicon.

8. Fluid detector according to one of claims 1 to 7, wherein the second semiconducting type material is a p-type metal oxide or a n-type metal oxide.

9. Fluid detector according to claim 8, wherein the p-type or n-type metal oxide is one of the group of materials consisting of the following materials:
- n-ZnO,
- n-TiO₂,
- n-SnO₂,
- n-WO₃,
- n-NbOx,
- n-VO_{X},
- n-In₂O₃,
- n-Fe₂O₃,
- p-NiO, and
- p-CuO.

10. Fluid detector according to one of claims 1 to 9, wherein the semiconducting nanoparticles (28) are made of one of the group of materials consisting of the following materials:
- CdS,
- CdSe,
- CdTe,
- PbS,
- InP,
- SnS,
- SnS₂,
- Bi₂S₃ and
- CuO.

11. Fluid detector according to one of claims 1 to 10, wherein the fluid detector (10) comprises a plurality of semiconductor detector cells (12), said semiconductor detector cells (12) being electrically interconnected in parallel and/or in series with each other.

12. Fluid detector according to claims 1 to 11, wherein the fluid detector (10) further comprises electrodes (36, 60) for contacting the at least one semiconductor detector cell (12).

13. Fluid detector according to claims 1 to 12, wherein the fluid detector (10) further comprises a measuring device (58) for measuring a voltage and/or a current of the at least one semiconductor detector cell (12).

14. Method for detecting fluids (32) by means of a fluid detector (10) comprising at least one semiconductor detector cell (12) including a photovoltaic semiconductor device (14) with at least one portion (16) made of a first semiconducting type material and at least one other portion (18) made of a second semiconducting type material, wherein the first and second semiconducting type materials differ in their charge carrier types and/or the density of their charge carriers and wherein the different portions (16, 18) adjoin each other with the formation of a junction between said different charge carrier types and/or charge carrier densities; and including a system for photo-induced electron transfer, the system comprising a plurality of semiconducting nanoparticles (28) on at least one surface area (30) of the portion (16) made of the second semiconducting type material, which surface area is accessible for the fluid (32), wherein the method comprises the steps:
- illuminating the fluid detector (10) with light having a photon energy in and/or below the near UV range and
- measuring a voltage and/or a current of the illuminated semiconductor detector cell (12).
